Europäisches Patentamt

European Patent Office

Office européen des brevets

(19)

(11) Veröffentlichungsnummer : **0 185 691**
**B1**

(12) **EUROPÄISCHE PATENTSCHRIFT**

(45) Veröffentlichungstag der Patentschrift :
**22.02.89**

(21) Anmeldenummer : **85902480.4**

(22) Anmeldetag : **30.05.85**

(86) Internationale Anmeldenummer :
**PCT/DE 85/00191**

(87) Internationale Veröffentlichungsnummer :
**WO/8505633 (19.12.85 Gazette 85/27)**

(51) Int. Cl.⁴ : **C 12 P 17/10** // (C12P17/10,
C12R1:645)

(54) VERFAHREN ZUR HERSTELLUNG VON CHANOCLAVIN.

(30) Priorität : **01.06.84 DE 3420954**

(43) Veröffentlichungstag der Anmeldung :
**02.07.86 Patentblatt 86/27**

(45) Bekanntmachung des Hinweises auf die Patenterteilung : **22.02.89 Patentblatt 89/08**

(84) Benannte Vertragsstaaten :
**AT BE CH DE FR GB IT LI LU NL SE**

(56) Entgegenhaltungen :
GB--A-- 1 170 600
Chemical Abstracts, Band 93, Nr. 25, 22. Dezember
1980, p. 486, Zusammenfassung 235207g, Columbus,
Ohio, US, W. Maier et al.: "Mutational biosynthesis in
a strain of Claviceps purpurea"
Chemical Abstracts, Band 96, Nr. 21, 24. Mai 1982, p.
558, Zusammenfassung 179355b, Columbus, Ohio,
US, L. Slokoska et al.: "Study of the strain Claviceps
sp. CPII which produces alkaloids. I. Relationship
between various stages of development and alkaloid
accumulation"
Chemical Abstracts, Band 84, Nr. 3, 19. Januar 1976,
p. 327, Zusammenfassung 15646y, Columbus, Ohio,
US, I. Grigorov et al.: "Obtaining some alkaloids from
Claviceps strain 214"
Chemical Abstracts, Band 97, Nr. 11, 13. September
1982, p. 408, Zusammenfassung 88317v, Columbus,
Ohio, US, K.K. Janardhanan et al.: "Studies on
Claviceps parasitic on Panicum species in India"

(73) Patentinhaber : **SCHERING AKTIENGESELLSCHAFT**
**Müllerstrasse 170/178**
**D-1000 Berlin 65 (DE)**

(72) Erfinder : **WILKE, Detief**
**Landwehr 6**
**D-3015 Wenningsen/Deister (DE)**
Erfinder : **WEBER, Alfred**
**Schützallee 56**
**D-1000 Berlin 37 (DE)**

**Beschreibung**

Die Erfindung betrifft das in dem Patentanspruch gekennzeichnete Verfahren. Chanoclavin = 2-Methyl-3-[1,3,4,5-tetrahydro-4-(methylamino)-benz (cd)-indol-5-yl]-2-propen-1-ol ist bekanntlich eine Vorstufe zur Biosynthese pharmakologisch wirksamer Ergotalkaloide und kann unter anderem zur Herstellung pharmakologisch wirksamer Verbindungen verwendet werden. (J. Med. Chem., 17, 1974, 300).

Es ist bekannt, daß man durch Kultivierung von Mikroorganismen der Gattung Claviceps Gemische von Ergotalkaloiden herstellen kann, die auch Chanoclavin enthalten (GB-A 1 170 600), die Auftrennung dieser Gemische ist aber recht aufwendig und die erzielten Ausbeuten an reinem Chanoclavin ist gering.

Es wurde gefunden, daß ein Pilzstamm Claviceps spec. Chanoclavin in hohen Ausbeuten und praktisch frei von anderen Ergotalkaloiden in das Kulturmedium ausscheidet. Dieser Stamm Claviceps spec. wurde aus einem Mutterkorn einer wild wachsenden Trespe (Bromus) isoliert. Er hat die interne Bezeichnung SCHERING, MBCE 373113 und ist bei der deutschen Sammlung für Mikroorganismen unter der Nummer DSM 2837 hinterlegt. Der Stamm bildet auf Agarmedien mit Glucose oder Saccharose als Kohlenstoffquelle und Asparagin, Ammoniumsuccinat oder Ammoniumcitrat als Stickstoffquelle kompakte, stark gefaltete, weiße im Alter rötliche Kolonien die aus Hyphen bestehen, die im Alter stark lichtbrechende Vakuolen aufweisen.

Die Hyphen haben einen Durchmesser von 3 bis 4 μm. Konidien werden nicht gebildet. Der Koloniendurchmesser beträgt nach 10 Tagen Kulturzeit 3 bis 4 cm und nach 20 Tagen Kulturzeit 6 bis 7 cm.

Das erfindungsgemäße Verfahren wird unter Bedingungen durchgeführt, die man üblicherweise zur Anzucht von Pilzkulturen für eine Stoffwechselsynthese anwendet. So werden zunächst in allgemein üblichen Vorversuchen die günstigsten Fermentationsbedingungen, wie z. B. die Auswahl des günstigsten Nährmediums, der technischen Bedingungen wie Temperatur, Belüftung pH-Wert und der optimalen Zeiten für die Germination und die Entwicklung des Mikroorganismus ermittelt.

Als Kohlenstoffquelle kann für das Fermentationsmedium beispielsweise Glucose oder Saccharose verwendet werden. Als Stickstoffquelle dient unter anderem Asparagin, Ammoniumsuccinat oder Ammoniumcitrat. Ferner enthält das Medium die nötigen Wuchsstoffe (beispielsweise Hefeextrakt) und Mineralstoffe (Kalium-, Magnesium-, Kalzium-, Eisen- und Zink-Kationen, sowie Sulfat, Phosphat und Nitrat-Anionen in der üblicherweise angewendeten Konzentration.

Die Fermentation kann ein- oder zweistufig erfolgen, wobei das für die Vorkultur verwendete Medium mit dem der Hauptkultur identisch sein oder von diesem verschieden sein kann. Anzucht und Vermehrung erfolgen vorzugsweise bei einer niederen Konzentration an Kohlenstoffquelle (10 bis 100 g/l), die Chanoclavin-Erzeugung selbst vorzugsweise bei höherer Konzentration an Kohlenstoffquelle (100 bis 300 g), wobei als Kohlenstoffquelle vorzugsweise Glucose verwendet wird.

Zu Beginn der Fermentation wird der pH-Wert des Mediums vorzugsweise in einem Bereich von 4 bis 6 eingestellt. Die Züchtungstemperatur liegt im Bereich von etwa 10 bis 35 °C vorzugsweise im Bereich von 20 bis 30 °C. Die Kulturbedingungen sind streng aerob. Die optimale Fermentationszeit wird durch Analyse des gebildeten Chanoclavins in üblicher Weise ermittelt.

Nach erfolgter Fermentation wird das gebildete Festuclavin in an sich bekannter Weise isoliert, beispielsweise indem man die Fermentationsansätze mit einem nicht mit Wasser mischbaren organischen Lösungsmittel, wie Ethylacetat, Methylisobutylketon, Dichlormethan, Chloroform oder Tetrachlorethan extrahiert, die Extrakte einengt und das erhaltene Rohprodukt durch Chromatographie und/oder Kristallisation reinigt.

Das nachfolgende Ausführungsbeispiel dient zur Erläuterung des erfindungsgemäßen Verfahrens.

Beispiel

Claviceps spec. DSM 2837 wird auf einem Nährmedium angezüchtet, welches folgende Komponenten enthält :

Saccharose (100 g/l), Citronensäure (10 g/l), Hefeextrakt (0,1 g/l), Kaliumhydrogenphosphat (500 mg/l), Magnesiumsulfat-Heptahydrat (300 mg/l), Ammoniumsulfat (69 mg/l), Calciumnitrat-Tetrahydrat (1 g/l), Eisensulfat-Heptahydrat (7 mg/l), Zinksulfat-Heptahydrat (6 mg/l). Das Nährmedium ist auf pH 5,1 eingestellt. Die Anzuchtskultur wird 14 bis 28 Tage lang bei 30 °C im Brutschrank aufbewahrt.

Ein ca. 1 cm² großes Mycelstück wird mittels eines Ultraturrax unter sterilen Bedingungen in 5 ml physiologischer Kochsalzlösung zerkleinert und damit 50 ml eine Vorkultur enthaltend Glucose (50 g/l), Citronensäure (7,5 g/l), Kaliumdihydrogenphosphat (500 mg/l), Magnesiumsulfat-Heptahydrat (300 mg/l), Ammoniumnitrat (6 g/l), Calziumnitrat-Tetrahydrat (1 g/l), Zinksulfat-Heptahydrat (6 mg/l), Eisen (II)-sulfat-Heptahydrat (7 mg/l), Hefeextrakt (100 mg/l) — eingestellt auf pH 5,1 — die sich in einem 500 ml Erlenmeyerkolben befindet, beimpft und auf einem Rundschüttler 4 Tage lang bei 24 °C und 220 Umdrehungen pro Minute kultiviert.

5 ml der so erhaltenen Vorkultur werden in 50 ml eines Mediums enthaltend Saccharose (200 g/l), Citronensäure (10 g/l), Hefeextrakt (0,1 g/l), Kaliumdihydrogenphosphat (500 mg/l), Magnesiumsulfat-Heptahydrat (300 mg/l), Ammoniumsulfat (6 g/l), Calciumnitrat-Tetrahydrat (1 g/l),

Eisensulfat-Heptahydrat (7 mg/l) und Zinksulfat-Heptahydrat (6 mg/l) — eingestellt auf pH 5,1 — die sich in einem 500 ml Erlenmeyerkolben befindet, überführt und 9 Tage lang bei 24 °C auf einem Rundschüttler mit 240 Umdrehungen pro Minute geschüttelt.

Dann wird das Kulturmedium abfiltriert und der Gehalt an Chanoclavin mittels der van Urk's-Reaktion (Mikrochim. Acta, 1959, 619-630) fotometrisch ermittelt. Die Konzentration des Kulturfiltrates beträgt 390 mg/l. Dünnschichtchromatographisch sind keine weiteren Ergotalkaloide nachweisbar.

**Patentanspruch**

Verfahren zur Herstellung von Chanoclavin, dadurch gekennzeichnet, daß man den Mikroorganismus Claviceps spec. DSM 2837 kultiviert und das gebildete Chanoclavin nach Beendigung der Fermentation isoliert.

**Claim**

Process for the preparation of chanoclavin, characterised in that the micro-organism Claviceps spec. DSM 2837 is cultivated and the chanoclavin formed is isolated after completion of fermentation.

**Revendication**

Procédé de préparation de chanoclavine, procédé caractérisé en ce que l'on cultive le micro-organisme Claviceps spec. DSM 2837 et on isole la chanoclavine formée après la fermentation.